# EUROPÄISCHE PATENTSCHRIFT

(11) **EP 0 595 166 B1**
(45) Veröffentlichungstag und Bekanntmachung des Hinweises auf die Patenterteilung: **21.05.1997**
(21) Anmeldenummer: 93116864.5
(22) Anmeldetag: 19.10.1993
(51) Int. Cl.: C07D 401/04, C07D 409/04, A61K 31/44

(54) **4-Heterocyclyl substituierte Dihydropyridine**
4-Heterocycle substituted dihydropyridines
4-Hétérocycliques dérivés de dihydropyridine

(30) Priorität: 30.10.1992 DE 4236707
(43) Veröffentlichungstag der Anmeldung: 04.05.1994
(73) Patentinhaber: BAYER AG, 51368 Leverkusen (DE)
(72) Erfinder: Junge, Bodo, Dr., D-42399 Wuppertal (DE); Hartwig, Wolfgang, Dr., D-42113 Wuppertal (DE); Meier, Heinrich, Dr., D-42105 Wuppertal (DE); Schohe-Loop, Rudolf, Dr., D-42327 Wuppertal (DE); Gao, Zhan, Dr., Bayer Beijing L. Of., Un.3009 30/F, Beijing 100020 Pr (CN); Schmidt, Bernard, Dr., D-51789 Lindlar (DE); de Jonge, Maarten, Dr., D-51491 Overath (DE); Schuurman, Teunis, Dr., D-53797 Lohmar (DE)

(56) Entgegenhaltungen:
- EP-A- 0 026 317
- EP-A- 0 088 276
- EP-A- 0 239 186
- DE-A- 1 670 824
- DE-A- 2 117 571
- DE-A- 2 635 916
- DE-A- 2 847 236
- DE-A- 3 445 356
- DE-A- 3 816 361
- GB-A- 1 129 158
- PATENT ABSTRACTS OF JAPAN vol. 015, no. 425 (C-0879)29. Oktober 1991 3,5-pyridinedicarboxylic acid, 1,4-dihydro-2,6-dimethyl-4-(2-thienyl)-,2-cyanoethyl methyl ester, RN=137347-41-2 "
- CHEMICAL ABSTRACTS, vol. 107, 1987, Columbus, Ohio, US; abstract no. 58891k, 'Synthesis of new 1,6-naphthyridines by aminomethynylation of 1,4-dihydropyrdines'
- CHEMICAL ABSTRACTS, vol. 108, 1988, Columbus, Ohio, US; abstract no. 55919k, 'A new condensed triheterocyclic system.' Seite 711 ;
- CHEMICAL ABSTRACTS, vol. 111, 1989, Columbus, Ohio, US; abstract no. 23355u, 'Enantioselective Hantzsch dihydropyridine synthesis via metalated chiral alkyl acetoacetate hydrazones' Seite 601 ;
- EUR. J. MED. CHEM.-CHIM. THER., Bd. 18, Nr. 2, 1983, Seiten 155 - 161 'Sila- pharmaca, xxv'
- J. HETEROCYCLIC CHEM. Bd. 25, Nr. 1, 1988, Seiten 81 - 87 'Directed lithiation of 4-halopyridines'
- CHEMICAL ABSTRACTS, vol. 91, 1979, Columbus, Ohio, US; abstract no. 56780a, 'Transesterification of esters of 2,6-dimethyl-1,4-dihydropyrid ine-3,5- dicarboxylic acids' Seite 686 ;
- CHEMICAL ABSTRACTS, vol. 83, 1975, Columbus, Ohio, US; abstract no. 28058n, '4-substituted 2,6-di methyl-3,5-di-beta-alkoxyethoxycaronyl-1,4 - dihydropyridines' Seite 493 ;
- TRENDS PHARMACOL. SCI. Bd. 11, Nr. 8, 1990, Seiten 309 - 310 'Nimodipine and the recovery of memory'

## Beschreibung

Die vorliegende Erfindung betrifft 4-Heterocyclyl substituierte Dihydropyridine, Verfahren zu ihrer Herstellung und ihre Verwendung in Arzneimitteln, insbesondere als Mittel zur Behandlung von Erkrankungen des zentralen Nervensystems.

Die Verbindung Nimodipin und ihre cerebrale Wirksamkeit ist bekannt (vgl. DOS 28 15 578). Außerdem sind 4-Heterocyclyl substituierte Dihydropyridine mit einer blutdrucksenkenden Wirkung (vgl. JP 239 353; DE 38 16 361-A; EP 88 276 A; DE 32 39 273-A; EP-A-0 026 317; WO 84/02 132-A; US 3 488 359; US 3 574 843) bekannt.

Die vorliegende Erfindung betrifft neue 4-Heterocyclyl substituierte Dihydropyridine aus der Gruppe
4-(2-Chlorthien-3-yl)-1,4-dihydro-2,6-dimethylpyridin-3,5-dicarbonsäure-isopropyl-2-methoxyethyl-ester
4-(2-Chlorthien-3-yl)-1,4-dihydro-2,6-dimethylpyridin-3,5-dicarbonsäure-cyclopentyl-2-methoxyethyl-ester
1,4-Dihydro-2,6-dimethyl-(4-(thien-3-yl)-pyridin-3,5-dicarbonsäurecyclopentyl-2-methoxyethyl-ester
1,4-Dihydro-2,6-dimethyl-(4-(thien-3-yl)-pyridin-3,5-isopropyl-2-methoxyethyl-ester
1,4-Dihydro-2,6-dimethyl-(4-(thien-3-yl)-pyridin-3,5-carbonsäureisopropyl-2-methoxyethyl-ester
1,4-Dihydro-2,6-dimethyl-(4-(thien-2-yl)-pyridin-3,5-dicarbonsäurecyclopentyl-2-methoxyethyl-ester
(+)-4-(2-Chlorthien-3-yl)-1,4-didydro-2,6-dimethyl-pyridin-3,6-dicarbonsäure-isopropyl-2-methoxyethyl-ester
(-)-4-(2-Chlorthien-3-yl)-1,4-didydro-2,6-dimethyl-pyridin-3,6-dicarbonsäure-isopropyl-2-methoxyethyl-ester.

Die erfindungsgemäßen Verbindungen existieren in stereoisomeren Formen, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen (vgl. E.L. Eliel, Stereochemistry of Carbon Compounds, McGraw Hill, 1962).

Außerdem wurden Verfahren zur Herstellung der erfindungsgemäßen Verbindungen gefunden, dadurch gekennzeichnet, daß man
[A] Aldehyde der allgemeinen Formel (II) 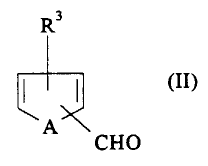 in welcher
   - A: für ein Schwefelatom steht, und
   - R³: für Wasserstoff oder Chlor steht,
   zunächst mit Acetessigsäureestern der allgemeinen Formel III

   H₃C-CO-CH₂-CO₂R² (III)

   in welcher
   R² für den Methoxyethylesterrest steht,
   gegebenenfalls unter Isolierung der Benzylidenverbindung umsetzt und anschliessend mit 3-Aminocrotonsäureestern der allgemeinen Formel IV 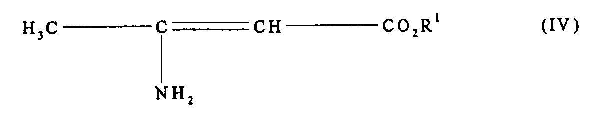 in welcher
   - R¹: für Isopropyl oder Cyclopentyl steht,
   in inerten Lösemitteln in Anwesenheit einer Base und ggf. Säuren umsetzt, oder
[B] Verbindungen der allgemeinen Formel V 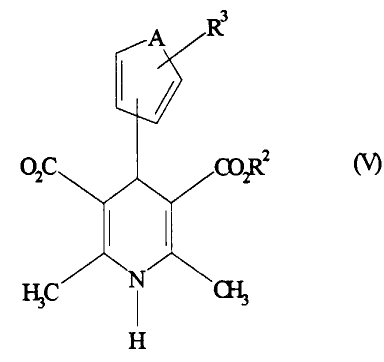 in welcher
   A, R² und R³ die oben angegebene Bedeutung haben,
   ggf. über ein reaktives Säurederivat, in Anwesenheit einer Base mit den entsprechenden Alkoholen umsetzt, wobei durch Einsatz der enantiomerenreinen Derivate der Verbindungen der allgemeinen Formel V die entsprechenden Enantiomeren der allgemeinen Formel I erhalten werden.

Die erfindungsgemäßen Verfahren können durch folgendes Formelschema beispielhaft erläutert werden:

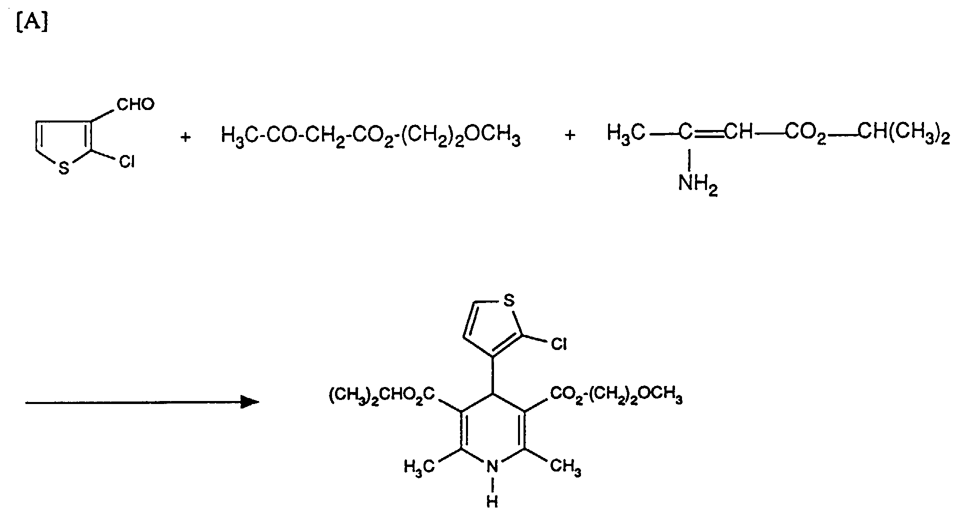

Als Lösemittel für das Verfahren [A] eignen sich hierbei alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldirnethylether, oder Diethylenglykoldimethylether, Acetonitril, oder Amide wie Hexamethylphosphorsäuretriamid oder Dimethylformamid, oder Essigsäure oder halogenierte Kohlenwasserstoffe wie Methylenchlorid, Tetrachlorkohlenstoff oder Kohlenwasserstoffe wie Benzol oder Toluol. Ebenso ist es möglich, Gemische der genannten Lösemittel zu verwenden. Bevorzugt sind Isopropanol, Ethanol, Tetrahydrofuran, Methanol, Dioxan und Dimethylformamid.

Als Lösemittel für das Verfahren [B] eignen sich die oben aufgeführten Lösemittel mit Ausnahme der Alkohole.

Als Basen eignen sich im allgemeinen Alkalihydride- oder alkoholate, wie beispielsweise Natriumhydrid oder Kalium-tert.butylat, oder cyclische Amine, wie beispielsweise Piperidin, Dimethylaminopyridin oder C₁-C₄-Alkylamine, wie beispielsweise Triethylamin. Bevorzugt sind in Abhängigkeit der jeweiligen Reaktionsschritte Piperidin, Dimethylaminopyridin, Pyridin, Natriumhydrid und Kalium-tert.butylat.

Im Fall des Verfahrens [A] eigenen sich als Säuren organische C₁-C₃-Carbonsäuren, wie beispielsweise Essigsäure oder Propionsäure. Bevorzugt ist Essigsäure.

Bei der Durchführung der erfindungsgemäßen Verfahren ist das Verhältnis der an der Reaktion beteiligten Stoffe beliebig. Im allgemeinen arbeitet man jedoch bei molaren Mengen der Reaktanden.

Die Reaktionstemperaturen können in einem größeren Bereich variiert werden. Im allgemeinen arbeitet man zwischen +10°C und +150°C, vorzugsweise zwischen +20°C und +100°C, insbesondere bei der Siedetemperatur des jeweiligen Lösemittels.

Die Umsetzungen können bei Normaldruck, aber auch bei erhöhtem oder erniedrigtem Druck (z.B. 0,5 bis 3 bar) durchgeführt werden. Im allgemeinen arbeitet man bei Normaldruck.

Einige Reaktionsschritte werden unter Schutzgasatmosphäre umgesetzt.

Zur Aktivierung der Carbonsäure eignen sich die üblichen Reagenzien wie anorganische Halogenide, beispielsweise Thionylchlorid, Phosphortrichlorid oder Phosphorpentachlorid, oder Carbonyldiimidazol, Carbodiimide wie Cyclohexylcarbodiimid oder 1-Cyclohexyl-3-[2-(N-methylmorpholino)ethyl]-carbodiimid-p-toluolsulfonat oder N-Hydroxyphthalimid oder N-Hydroxy-benztriazol.

Enantiomerenreine Formen erhält man z.B., indem man Diastereomerengemische der Verbindungen der allgemeinen Formel (I), in welcher R² oder R³ für einen optisch aktiven Esterrest steht, nach üblicher Methode trennt, anschließend entweder direkt umestert oder zuerst die chiralen Carbonsäuren herstellt und dann durch Veresterung die enantiomerenreinen Dihydropyridine herstellt.

Geeignet als chirale Esterreste sind alle Ester enantiomerenreiner Alkohole wie beispielsweise Phenylethanol, Milchsäure, Milchsäureester, Mandelsäure, Mandelsäureester, 2-Aminoalkohole, Zuckerderivate, Hydroxyaminosäurederivate und viele andere enantiomerenreine Alkohole mehr.

Die Trennung der Diastereomeren erfolgt im allgemeinen entweder durch fraktionierte Kristallisation, durch Säulenchromatographie oder durch Craig-Verteilung. Welches das optimale Verfahren ist, muß von Fall zu Fall entschieden werden, manchmal ist es auch zweckmäßig, Kombinationen der einzelnen Verfahren zu benutzen. Besonders geeignet ist die Trennung durch Kristallisation oder Craig-Verteilung bzw. eine Kombination beider Verfahren.

Die enantiomerenreinen Verbindungen sind auch zugänglich durch Chromatographie der racemischen Ester auf chiralen Phasen

Die Verbindungen der allgemeinen Formel (II) sind an sich bekannt oder können nach bekannten Verfahren hergestellt werden.

Die Acetessigsäurederivate der allgemeinen Formel (III) sind bekannt oder können nach bekannten Verfahren hergestellt werden.

Die Aminocrotonsäurederivate der Formel (IV) sind bekannt oder können nach bekannten Verfahren hergestellt werden.

Die Verbindungen der allgemeinen Formel (V) sind neu und können hergestellt werden, indem man Verbindungen der allgemeinen Formel (II) zunächst mit Acetessigestem der allgemeinen Formel (III) und anschließend mit Verbindungen der allgemeinen Formel (VI) 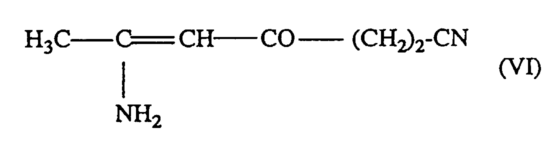 wie unter Verfahren [A] beschrieben versetzt, und den entstandenen Dihydropyridincyanoethylester mit einer der oben aufgeführten Basen, vorzugsweise Natriumhydroxid und Kalium-tert.butylat, behandelt.

Die vorstehende Herstellungsverfahren sind lediglich zur Verdeutlichung angegeben. Die Herstellung der Verbindungen der allgemeinen Formel (I) ist nicht auf diese Verfahren beschränkt, sondern jede Modifikation dieser Verfahren ist in gleicher Weise für die Herstellung der erfindungsgemäßen Verbindungen anwendbar.

Die erfindungsgemäßen Verbindungen zeigen ein nicht vorhersehbares, wertvolles pharmakologisches Wirkspektrum.

Die erfindungsgemäßen Verbindungen sind Calciumkanalliganden mit Selektivität für L-Typ-Calciumkanäle des zentralen Nervensystems. Diese Selektivität läßt sich z.B. durch Vergleich der Bindungsaffinitäten zu DHP-Bindungstellen an Rattenhirn und Rattenherz zeigen.

Die Verbindungen beeinflussen positiv Lern- und Gedächtnisleistungen, wie ihre leistungsverbessernde Wirkung auf Ratten in typischen Lern- und Gedächtnismodellen wie Wasser-Labyrinth, Morris-Labyrinth, Passives Vermeiden, Reminiszenztests in automatisierten Skinner-Boxen demonstriert.Sie besitzen ein antidepressives Potential, wie ihre Wirksamkeit im Rattenschwimmtest nach Porsolt belegt.

### Bindungsassays:

Die Bindungsaffinitäten zu PN-200-110-Bindungsstellen im Rattenhirnen bzw. Rattenherzen werden nach Rampe D.R., Mutledge, Janis R.A., Triggle D.J.: Can. Journ. Physiol. Pharmacol. 65, (1987) 1452 bestimmt.

### Wasserlabyrinth:

Alte Wistar-Ratten (24 Monate) werden an die Startposition in einem mit kaltem (14-15°) Wasser gefüllten, durch senkrechte Barrieren unterteilten Plastiktank (120x50x40 cm) gesetzt Um zu einer Leiter zu gelangen, die den Tieren das Entkommen aus dem Wasser ermöglicht, müssen sie um diese Barrieren herumschwimmen. Die Zeit, die zum Auffinden des Ausstiegs benötigt wird und die Zahl der Fehler auf dem Weg dorthin werden aufgezeichnet Dabei wird ein Fehler definiert als Schwimmen in eine Sackgasse oder Schwimmen über die Grenzlinie imaginärer Quadrate, in die der Tank unterteilt ist, in die Richtung fort vom Ausstieg.
Die Ratten bleiben bis zum Finden des Ausgangs, längstens aber 300 sec. im Labyrinth. Dann werden sie aufgenommen, getrocknet und unter einem Rotlicht gewärmt Danach kehren sie in ihre Heimatkäfige zurück.
In einem typischen Experiment werden durch einen Vortest zwei äquivalente Tiergruppen (Placebo, Testsubstanz je n = 15) ermittelt. Anschließend durchlaufen die Tiere 6 Testsitzungen, zwei je Tag. Testsubstanzen bzw. Placebo werden 30 min. vor den Versuchen per os verabreicht. Maß für die lern- und gedächtnisverbessernde Wirkung der Testsubstanzen im Vergleich zu Placebo sind Verkürzung der Zeit bis zum Erreichen des Ausstiegs, Verringerung der Zahl der Fehler und Vergrößerung der Zahl der Tiere,die den Ausstieg überhaupt finden.

### Rattenschwimmtest nach Porsolt

Während eines Vortests werden junge Ratten in einem Glaszylinder (40 xm hoch, 20 cm Durchmesser) gesetzt, der 17 cm hoch mit Wasser von 25°C gefüllt ist. Nach 20 min im Wasser werden die Tiere herausgenommen und unter einer Lampe 30 min gewärmt. In diesem Vortest versuchen alle Ratten, aus dem Zylinder zu entkommen, bis sie nach etwa 15 min immobil verharren ("behavioral dispair", Aufgabeverhalten). 24 h später beginnt die Testsitzung in der die Ratten wie am Vortag in den Glaszylinder gesetzt werden, jedoch diesmal nur 5 min. Die Zeitspannen, die die Ratten während dieser 5 min immobil verharren, werden aufgezeichnet. Dabei wird eine Ratte als immobil angesehen, die aufrecht im Wasser treibend nur noch minimale Bewegungen ausführt, um den Kopf über Wasser zu halten. Placebo bzw. Testsubstanzen (0,25, 0,5, 1, 5, 10 mg/kg; n = 6 je Gruppe) werden dreimal per os verabreicht: 23, 5 und 1 h vor der Testsitzung (1, 19, 23 h nach dem Vortest). Die antidepressive Wirkung der Testsubstanzen zeigt sich in der Reduktion der Immobilitätsdauer im Vergleich zu den Placebowerten.

Aufgrund ihrer pharmakologischen Eigenschaften können sie für die Herstellung von Arzneimitteln zur Behandlung von zentral degenerativen Erkrankungen eingesetzt werden, wie z.B. Auftreten bei Demenzen (Multiinfarktdemenz, MID, primär degenerativer Demenz PDD, prä- und seniler Alzheimerscher Krankheit, HIV-Demenz und andere Demenzformen), Parkinsonscher Krankheit oder amyotrophischer Lateralsklerose.

Weiterhin eignen sich die Wirkstoffe zur Behandlung von Hirnleistungsstörungen im Alter, des hirnorganischen Psychosyndroms (HOPS, Organic brain syndrom, OBS) und von altersbedingten Gedächtnisstörungen (age associated memory impairment, AAMI).

Sie sind wertvoll zur Prophylaxe und zur Bekämpfung der Folgen cerebraler Durchblutungsstörungen wie cerebraler Ischämien, Schlaganfällen und von Subarachnoidalblutungen und zur Behandlung von Hirntraumen.

Sie sind geeignet zur Behandlung von Depressionen. Weitere Anwendungsgebiete sind die Behandlung von Migräne, von Neuropathien, von Suchterkrankungen und Entzugserscheinungen.

Zur vorliegenden Erfindung gehören auch pharmazeutische Zubereitungen, die neben inerten, nicht-toxischen, pharmazeutisch geeigneten Hilfs- und Trägerstoffen eine oder mehrere Verbindungen der allgemeinen Formel (I) enthalten, oder die aus einem oder mehreren Wirkstoffen der Formel (I) bestehen, sowie Verfahren zur Herstellung dieser Zubereitungen.

Die Wirkstoffe der Formel (I) sollen in diesen Zubereitungen in einer Konzentration von 0,1 bis 99,5 Gew.-%, bevorzugt von 0,5 bis 95 Gew.-% der Gesamtmischung vorhanden sein.

Neben den Wirkstoffen der Formel (I) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Im allgemeinen hat es sich als vorteilhaft erwiesen, den oder die Wirkstoffe der Formel (I) in Gesamtmengen von etwa 0,01 bis etwa 100 mg/kg, bevorzugt in Gesamtmengen von etwa 1 mg/kg bis 50 mg/kg Körpergewicht je 24 Stunden, gegebenenfalls in Form mehrerer Einzelgaben, zur Erzielung des gewünschten Ergebnisses zu verabreichen.

Es kann aber gegebenenfalls vorteilhaft sein, von den genannten Mengen abzuweichen, und zwar in Abhängigkeit von der Art und vom Körpergewicht des behandelten Objekts, vom individuellen Verhalten gegenüber dem Medikament, der Art und Schwere der Erkrankung, der Art der Zubereitung und Applikation, sowie dem Zeitpunkt bzw. Intervall, zu welchem die Verabreichung erfolgt.

Die jeweils aufgeführten R_{f}-Werte wurden - sofern nicht anders vermerkt - durch Dünnschichtchromatographie auf Kieselgel (Alufolie, Kieselgel 60 F 254, Fa. E. Merck) ermittelt. Die Visualisierung der Substanzflecke geschah durch Betrachten unter UV-Licht und/oder durch Besprühen mit 1%iger Kaliumpermanganat-Lösung.

Die Flash-Chromatographie wurde auf Kieselgel 60, 0,040 - 0,064 mm, Fa. E. Merck, durchgeführt. Elution mit Lösemittelgradienten bedeutet: Beginnend mit der reinen, unpolaren Lösemittelgemischkomponente wird in einem steigendem Ausmaß die polare Laufmittelkomponente zugemischt, bis das gewünschte Produkt eluiert wird (DC-Kontrolle).

### Herstellungsbeispiele

### Beispiel 1

### 4-(2-Chlorthien-3-yl)-1,4-dihydro-2,6-dimethyl-pyridin-3-(2-methoxyethyl) -5-(2-cyanoethyl)-diester

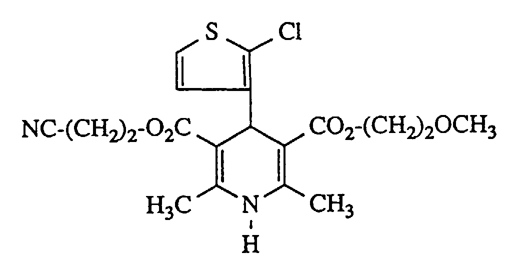

14,7 g (100 mmol) 2-Chlorthiophen-3-aldehyd, 16,0 g (100 mmol) Acetessigsäure-(2-methoxyethyl)-ester und 15,4 g (100 mmol) 2-Aminocrotonsäure-(2-cyano-ethyl)-ester werden in 150 ml Isopropanol umgesetzt. Man erhält 23,1 g (54%) der Titelverbindung vom Schmp. 112-114°C, welches als Rohprodukt weiter umgesetzt wird.

### Weitere Umsetzung:

22,9 g (54 mmol) der Verbindung aus Beispiel 1 werden in 229 ml 1,2-Dimethoxyethan gelöst. Innerhalb von 30 min werden bei Raumtemperatur 72 ml In Natronlauge zugetropft und die Reaktionslösung 18 h bei Raumtemperatur gerührt. Anschließend werden 72 ml 1 n Salzsäure zugetopft und danach noch 85 ml Wasser, worauf langsame Kristallisation einsetzt Es wird noch 2 h auf 15°C gekühlt und das Kristallisat abgesaugt. Man erhält 9,6 g (48%) 4-(2-Chlorthien-3-yl)-1,4-dihydro-2,6-dimethyl-pyridin-3,5-dicarbonsäure-2-methoxyethyl-monoester vom Schmp. 157-159°C (Zers.). Aus der Mutterlauge erhält man nach Einengen auf das halbe Volumen weitere 8,5 g vom Schmp. 150-157°C.

### Beispiel 2

### 4-(2-Chlorthien-3 -yl)-1,4-dihydro-2,6-dimethyl-pyridin-3,5-dicarbonsäure-isopropyl-2-methoxyethylester

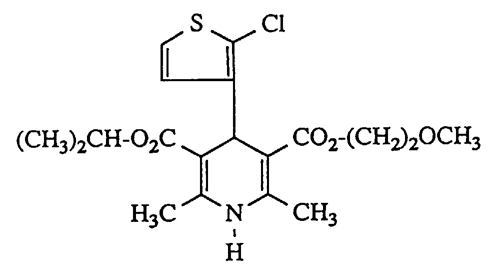

A)
   7,3 g (50 mmol) 2-Chlorthiophen-3-aldehyd werden mit 7,2 g (50 mmol) Acetessigsäureisopropylester in 75 ml Isopropanol gelöst Nach Zugabe von 0,35 ml (3,5 mmol) Piperidin und 0,20 ml (3,5 mmol) Essigsäure in 6 ml Isopropanol erwärmt man 2 h auf 40°C, gibt dann 8,0 g (50 mmol) 3-Aminocrotonsäure-2-methoxyethylester zu und rührte weitere 8 h bei 40°C. Nach Abkühlen rührt man den Ansatz in eine Mischung aus 400 ml Wasser und 200 ml Toluol ein. Die wäßrige Phase wird noch einmal mit 100 ml Toluol extrahiert und die vereinigten organischen Phasen mit je 100 ml 0,1 n Salzsäure und 1%iger Natriumhydrogencarbonatlösung, zweimal mit je 100 ml Wasser gewaschen und über Natriumsulfat getrocknet. Die nach Einengen auf ca. 75 ml, Filtration, weiterem Einengen, Ausfällen mit Petrolether und erneuter Filtration erhaltenen Rohproduktfraktionen werden durch Chromatographie an Kieselgel in Essigester/Cyclohexan 1:1 und Kristallisation aus Essigester / Cyclohexan gereinigt Man erhält 8,5 g (41%) der Zielverbindung vom Schmp. 125-126°C. Aus der Mutterlauge erhält man weitere 2,7 g (13%) vom gleichen Schmelzpunkt.
B)
   5,6 g (15 mmol) 4-(2-Chlorthiophen-3-yl)-1,4-dihydro-2,6-dimethyl-pyridin-3,5-dicarbonsäure-2-methoxyethylmonoester werden in 50 ml wasserfreiem Tetrahydrofuran auf 50°C erwärmt Man gibt 2,7 g (16,5 mmol) Carbonyldiimidazol, gelöst in 35 ml wasserfreiem Tetrahydrofuran zu und rührt ca 1 h bis zu Beendigung der Kohlendioxidentwicklung. Dann destilliert man unter Normaldurck 100 ml Lösemittel ab und gibt gleichzeitig 100 ml Isopropanol zu, wodurch eine Siedetemperatur von 82°C erreicht wird. Anschließend hält man noch 24 h am Sieden (dünnschichtchromatographische Kontrolle des Umsatzes in Cyclohexan / Essigester 30:70). Zur Aufarbeitung giebt man auf eine Mischung aus 400 ml 5%iger Natriumchloridlösung, 33 ml 1 N Salzsäure und 200 ml Toluol und extrahiert nach Trennung der Phasen die Wäßrige noch einmal mit Toluol. Die vereinigten organischen Phasen werden mit je 100 ml 0,1 N Salzsäure, Natriumhydrogencarbonatlösung und zweimal mit Wasser gewaschen, über Natriumsulfat getrocknet und auf ca. 50 ml eingeengt. Das nach Zugabe von 100 ml Petrolether ausgefällte Rohprodukt wird durch Chromatographie an Kieselgel in Cyclohexan / Essigester 1:1 und Kristallisation aus Cyclohexan / Essigester gereinigt. Man erhält so 3,9 g (63%) der Zielverbindung vom Schmelzpunkt 123-124°C.

In Analogie zur Vorschrift des Beispiels 2 werden die in der Tabelle 1 aufgeführten Verbindungen hergestellt:

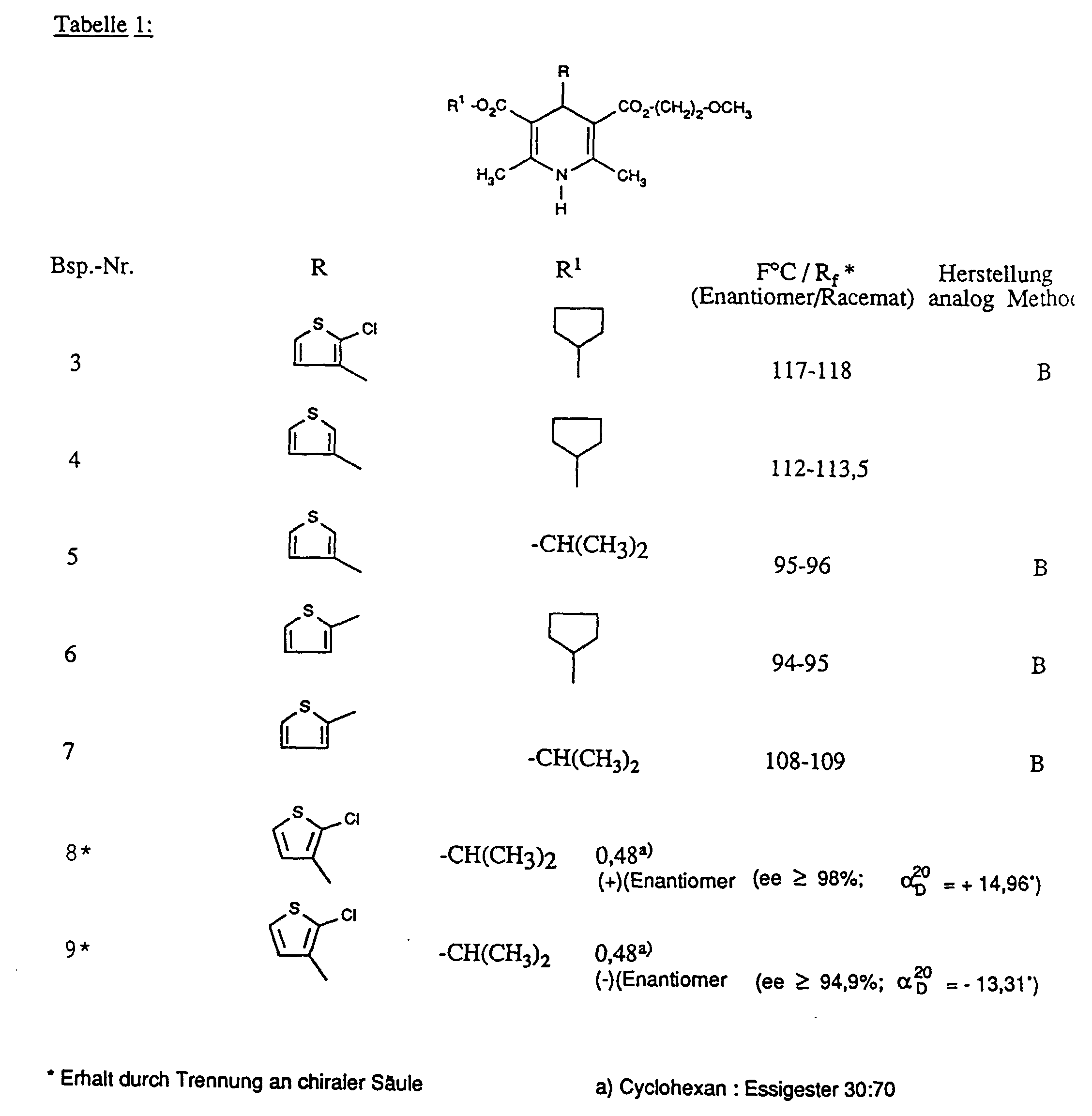

## Patentansprüche

1. 4-Heterocyclyl substituierte Dihydropyridine aus der Gruppe
4-(2-Chlorthien-3-yl)-1,4-dihydro-2,6-dimethylpyridin-3,5-dicarbonsäure-isopropyl-2-methoxyethyl-ester
4-(2-Chlorthien-3-yl)-1,4-dihydro-2,6-dimethylpyridin-3,5-dicarbonsäure-cyclopentyl-2-methoxyethyl-ester
1,4-Dihydro-2,6-dimethyl-(4-(thien-3-yl)-pyridin-3,5-dicarbonsäure-cyclopentyl-2-methoxyethyl-ester
1,4-Dihydro-2,6-dimethyl-(4-(thien-3-yl)-pyridin-3,5-isopropyl-2-methoxyethyl-ester
1,4-Dihydro-2,6-dimethyl-(4-(thien-3-yl)-pyridin-3,5-carbonsäure-isopropyl-2-methoxyethyl-ester
1,4-Dihydro-2,6-dimethyl-(4-(thien-2-yl)-pyridin-3,5-dicarbonsäure-cyclopentyl-2-methoxyethyl-ester
(+)-4-(2-Chlorthien-3-yl)-1,4-didydro-2,6-dimethyl-pyridin-3,6-dicarbonsäure-isopropyl-2-methoxyethyl-ester
(-)-4-(2-Chlorthien-3-yl)-1,4-didydro-2,6-dimethyl-pyridin-3,6-dicarbonsäure-isopropyl-2-methoxyethyl-ester.

2. Verfahren zur Herstellung von Heterocyclyl substituierten Dihydropyridinen gemäß Anspruch 1, dadurch gekennzeichnet, daß man
[A] Aldehyde der allgemeinen Formel II 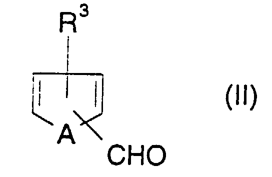 in welcher
A für ein Schwefelatom steht, und
R³ für Wasserstoff oder Chlor steht,
zunächst mit Acetessigsäureestern der allgemeinen Formel III
H₃C-CO-CH₂-CO₂R² (III)
in welcher
R² für den Methoxyethylesterrest steht,
gegebenenfalls unter Isolierung der Benzylidenverbindung umsetzt und anschließend mit 3-Aminocrotonsäureestern der allgemeinen Formel IV 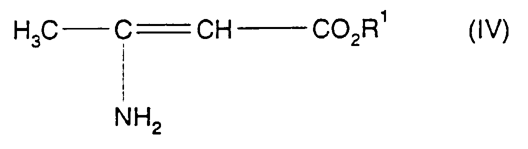 in welcher
R¹ für Isopropyl oder Cyclopentyl steht,
in interten Lösemitteln in Anwesenheit einer Base und ggf. Säuren umsetzt, oder
[B] Verbindungen der allgemeinen Formel V 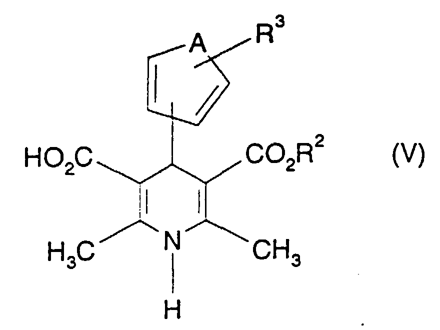 in welcher
A, R² und R³ die oben angegebenene Bedeutung haben,
ggf. über ein reaktives Säurederivat, in Anwesenheit einer Base mit den entsprechenden Alkoholen umsetzt, wobei durch Einsatz der enantiomerenreinen Derivate der Verbindungen der allgemeinen Formel V die entsprechenden Enantiomeren der allgemeinen Formel I erhalten werden.

3. Arzneimittel enthaltend mindestens ein 4-Heterocyclyl substituiertes Dihydropyridin gemäß Anspruch 1.

4. Verfahren zur Herstellung eines Arzneimittels gemäß Anspruch 3, dadurch gekennzeichnet, daß man mindestens ein 4-Heterocyclyl substituiertes Dihydropyridin ggf. mit Hilfe von geeigneten Hilfs- und Trägerstoffen in eine geeignete Applikationsform überführt.

5. Verwendung von 4-Heterocyclyl substituierten Dihydropyridinen gemäß Anspruch 1 zur Herstellung von Arzneimitteln zur Behandlung von cerebralen Erkrankungen.

## Claims

1. 4-Heterocyclyl-substituted dihydropyridines from the group
isopropyl 2-methoxyethyl 4- (2-chlorothien-3-yl)-1,4-dihydro-2,6-dimethylpyridine-3,5-dicarboxylate
cyclopentyl 2-methoxyethyl 4-(2-chlorothien-3-yl)-1,4-dihydro-2,6-dimethylpyridine-3,5-dicarboxylate
cyclopentyl 2-methoxyethyl 1,4-dihydro-2,6-dimethyl-4-(thien-3-yl)-pyridine-3,5-dicarboxylate
isopropyl 2-methoxyethyl 1,4-dihydro-2,6-dimethyl-4-(thien-3-yl)-pyridine-3,5-dicarboxylate
isopropyl 2-methoxyethyl 1,4-dihydro-2,6-dimethyl-4-(thien-3-yl)-pyridine-3,5-dicarboxylate
cyclopentyl 2-methoxyethyl 1,4-dihydro-2,6-dimethyl-4-(thien-2-yl)-pyridine-3,5-dicarboxylate
isopropyl 2-methoxyethyl (+) -4- (2-chlorothien-3-yl)-1,4-dihydro-2,6-dimethyl-pyridine-3,6-dicarboxylate
isopropyl 2-methoxyethyl (-) -4- (2-chlorothien-3-yl)-1,4-dihydro-2,6-dimethyl-pyridine-3,6-dicarboxylate.

2. Process for the preparation of heterocyclyl-substituted dihydropyridines according to Claim 1, characterised in that
[A] aldehydes of the general formula II 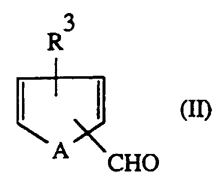 in which
A represents a sulphur atom, and
R³ represents hydrogen or chlorine,
are first reacted with acetoacetic acid esters of the general formula III
H₃C-CO-CH₂-CO₂R² (III)
in which
R² represents the methoxyethyl ester radical,
if appropriate with isolation of the benzylidene compound, and the products are then reacted with 3-aminocrotonic acid esters of the general formula IV 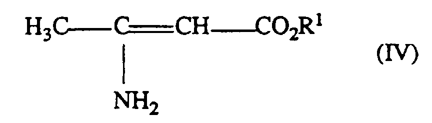 in which
R¹ represents isopropyl or cyclopentyl,
in inert solvents in the presence of a base and, if appropriate, acids, or
[B] compounds of the general formula V 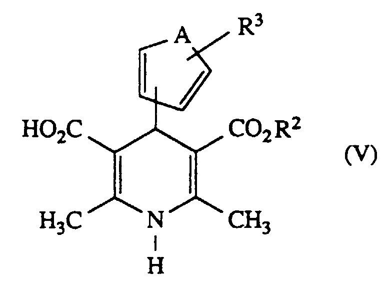 in which
A, R² and R³ have the abovementioned meaning,
are reacted with the appropriate alcohols, if appropriate via a reactive acid derivative, in the presence of a base, where, by use of the enantiomerically pure derivatives of the compounds of the general formula V, the corresponding enantiomers of the general formula I are obtained.

3. Medicament containing at least one 4-heterocyclyl-substituted dihydropyridine according to Claim 1.

4. Process for the production of a medicament according to Claim 3, characterised in that at least one 4-heterocyclyl-substituted dihydropyridine is converted into a suitable administration form, if appropriate with the aid of suitable auxiliaries and excipients.

5. Use of 4-heterocyclyl-substituted dihydropyridines according to Claim 1 for the production of medicaments for the treatment of cerebral disorders.

## Revendications

1. Dihydropyridines substituées par un groupe hétérocyclyle en position 4, choisies parmi le groupe comprenant les composés suivants:
ester isopropylique et 2-méthoxyéthylique de l'acide 4-(2-chlorothién-3-yl)-1,4-dihydro-2,6-diméthylpyridine-3,5-dicarboxylique,
ester cyclopentylique et 2-méthoxyéthylique de l'acide 4-(2-chlorothién-3-yl)-1,4-dihydro-2,6-diméthylpyridine-3,5-dicarboxylique,
ester cyclopentylique et 2-méthoxyéthylique de l'acide 1,4-dihydro-2,6-diméthyl-4-(thién-3-yl)-pyridine-3,5-dicarboxylique,
ester isopropylique et 2-méthoxyéthylique de 1,4-dihydro-2,6-diméthyl-4-(thién-3-yl)-pyridine en positions 3 et 5,
ester isopropylique et 2-méthoxyéthylique de l'acide 1,4-dihydro-2,6-diméthyl-4-(thién-3-yl)-pyridine-3,5-dicarboxylique,
ester isopropylique et 2-méthoxyéthylique de l'acide 1,4-dihydro-2,6-diméthyl-4-(thién-2-yl)-pyridine-3,5-dicarboxylique,
ester isopropylique et 2-méthoxyéthylique de l'acide (+)-4-(2-chlorothién-3-yl)-1,4-dihydro-2,6-diméthylpyridine-3,6-dicarboxylique,
ester isopropylique et 2-méthoxyéthylique de l'acide (-)-4-(2-chlorothién-3-yl)-1,4-dihydro-2,6-diméthylpyridine-3,6-dicarboxylique.

2. Procédé pour la préparation de dihydropyridines substituées par un groupe hétérocyclyle selon la revendication 1, caractérisé en ce que
[A] on fait réagir des aldéhydes répondant à la formule générale II 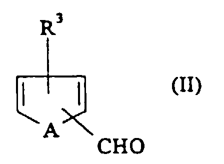 dans laquelle
A représente un atome de soufre et
R³ représente un atome d'hydrogène ou un atome de chlore,
d'abord avec des esters acétylacétiques répondant à la formule générale III
H₃C-CO-CH₂-CO₂R² (III)
dans laquelle
R² représente le radical d'ester méthoxyéthylique,
éventuellement avec isolation du composé de benzylidène, et
ensuite avec des esters 3-aminocrotoniques répondant à la formule générale IV 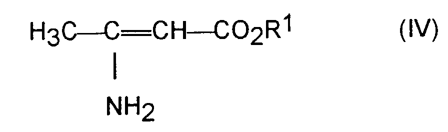 dans laquelle
R¹ représente un groupe isopropyle ou un groupe cyclopentyle,
dans des solvants inertes en présence d'une base et éventuellement d'acides, ou bien
[B] on fait réagir des composés répondant à la formule générale V 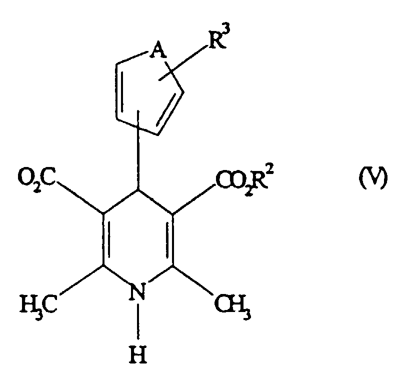 dans laquelle
A, R² et R³ ont la signification indiquée ci-dessus,
le cas échéant via un dérivé d'acide réactif, en présence d'une base, avec les alcools correspondants, dans lequel, par la mise en oeuvre des dérivés énantiomères, sous forme pure, des composés répondant à la formule générale (V), on obtient les énantiomères correspondants répondant à la formule générale (I).

3. Médicament contenant au moins une dihydropyridine substituée par un groupe hétérocyclyle en position 4 selon la revendication 1.

4. Procédé pour la préparation d'un médicament selon la revendication 3, caractérisé en ce qu'on transforme en une forme d'application appropriée au moins une dihydropyridine substituée par un groupe hétérocyclyle en position 4, le cas échéant à l'aide de substances auxiliaires et de support ou d'excipients appropriés.

5. Utilisation de dihydropyridines substituées par un groupe hétérocyclyle selon la revendication 1 pour la préparation de médicaments pour le traitement de maladies cérébrales.
